# EUROPÄISCHE PATENTSCHRIFT

(11) **EP 0 681 008 B1**
(45) Veröffentlichungstag und Bekanntmachung des Hinweises auf die Patenterteilung: **14.02.2001**
(21) Anmeldenummer: 95106240.5
(22) Anmeldetag: 26.04.1995
(51) Int. Cl.: C09B 67/22, C09B 62/527, D06P 1/38, C09B 62/085, C09B 62/44, C09B 67/00

(54) **Schwarze Farbstoffmischungen von faserreaktiven Azofarbstoffen und ihre Verwendung zum Färben von hydroxy- und/oder carbonamidgruppenhaltigem Fasermaterial**
Black mixtures of reactive azo dyes and their use for dyeing fibrous material containing hydroxy and/or carbonamide groups
Mélanges de colorants noirs de colorants azoiques réactifs et leur utilisation pour la teinture de matière fibreuse contenant des groupes hydroxyles et/ou carbonamides

(30) Priorität: 02.05.1994 DE 4415385
(43) Veröffentlichungstag der Anmeldung: 08.11.1995
(73) Patentinhaber: DyStar Textilfarben GmbH & Co. Deutschland KG, 60318 Frankfurt am Main (DE)
(72) Erfinder: Dannheim, Jörg, Dr., D-60489 Frankfurt am Main (DE); Russ, Werner Hubert, Dr., D-65439 Flörsheim (DE)

(56) Entgegenhaltungen:
- EP-A- 0 040 806
- EP-A- 0 111 830
- EP-A- 0 177 445
- EP-A- 0 545 219
- EP-A- 0 644 240
- CHEMICAL ABSTRACTS, vol. 114, no. 8, 25.Februar 1991 Columbus, Ohio, US; abstract no. 64186v, Seite 105; & JP-A-02 202 956 (REEWHA INDUSTRIAL CO) 13.August 1990

## Beschreibung

Die Erfindung liegt auf dem technischen Gebiet der faserreaktiven Farbstoffe.

Schwarzfärbende Farbstoffmischungen sind bereits aus den japanischen Patentanmeldungs-Veröffentlichungen Hei-2-073870 und Hei-2-202956 sowie aus den koreanischen Patentschriften Nrs. 91/2676, 91/6386 und 91/8343 bekannt, die sich zur Herstellung von tiefschwarzen Färbungen auf Cellulosefasermaterialien eignen. Diese bekannten Farbstoffmischungen weisen jedoch gewisse anwendungstechnische Mängel auf; so ist insbesondere ihre Auswaschbarkeit und ihre Naßliegeechtheit verbesserungsbedürftig.

Mit der vorliegenden Erfindung wurden Farbstoffmischungen gefunden, die einen oder mehrere, wie 2, 3 oder 4, Disazofarbstoffe entsprechend der allgemeinen Formel (1) und einen oder mehrere, wie 2, 3 oder 4, Monoazofarbstoffe entsprechend der allgemeinen Formel (2) enthalten.

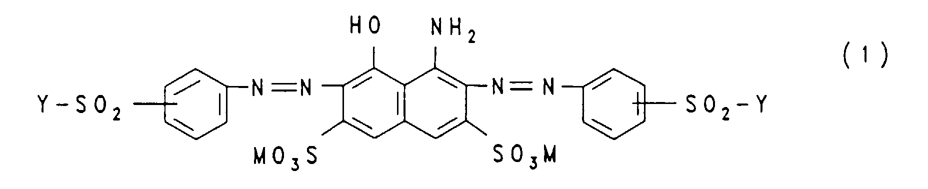

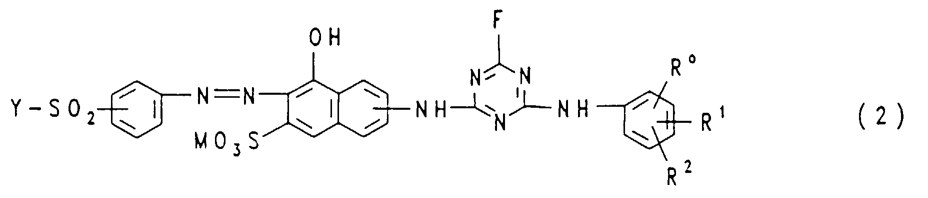

In diesen Formeln bedeuten:
- M: ist Wasserstoff oder ein Alkalimetall, wie Lithium, Natrium und Kalium;
- R^{o}: ist Sulfo oder Carboxy;
- R¹: ist Wasserstoff, Methyl, Ethyl, Methoxy, Ethoxy, Sulfo oder Carboxy, vorzugsweise Methoxy und Wasserstoff und insbesondere Wasserstoff;
- R²: ist Wasserstoff, Methyl, Ethyl, Methoxy oder Ethoxy, vorzugsweise Wasserstoff;
- Y: ist jedes, unabhängiges voneinander, Vinyl, β-Chlorethyl, β-Phosphatoethyl, β-Thiosulfatoethyl oder β-Sulfatoethyl, bevorzugt Vinyl und insbesondere β-Sulfatoethyl;
in Formel (2) ist die Fluortriazinylamino-Gruppe in 2- oder 3-Stellung an den 8-Hydroxy-6-sulfo-naphthalin-Rest gebunden.

Die einzelnen Formelglieder in den Formeln (1) und (2) und ebenso in den nachfolgend genannten allgemeinen Formeln können im Rahmen ihrer Definition zueinander gleiche oder voneinander verschiedene Bedeutungen besitzen.

Im allgemeinen sind der oder die Disazofarbstoffe der allgemeinen Formel (1) und der oder die Azofarbstoffe der allgemeinen Formel (2) in der Mischung in einem molaren Mischungsverhältnis von 1:0,3 bis 1:1,4 enthalten; bevorzugt liegt das Molverhältnis von (1):(2) bei 1:0,5 bis 1:1.

Die erfindungsgemäßen Farbstoffmischungen können zusätzlich als Nuancierkomponenten bis zu 10 Gew.-%, insbesondere bis 5 Gew.-%, bezogen auf die erfindungsgemäße Farbstoffmischung, an einem oder mehreren, wie 2, 3 oder 4, Monoazofarbstoffen entsprechend den allgemeinen Formeln (3a), (3b) und/oder (3c) 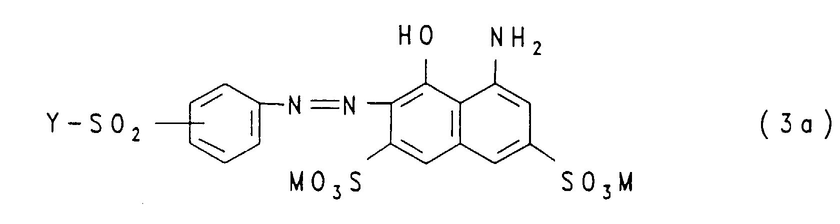 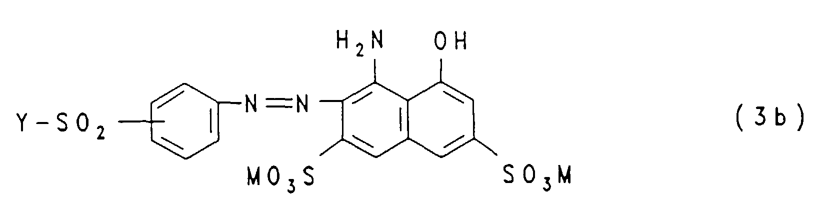 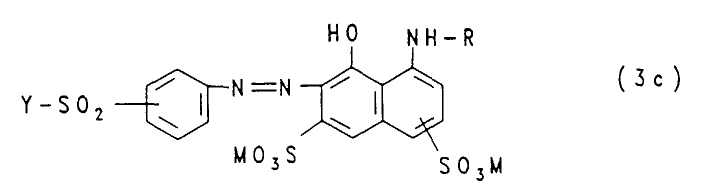 enthalten, in welchen M und Y eine der obengenannten Bedeutungen haben und die eine Gruppe -SO₃M in Formel (3c) in meta- oder para-Stellung zur Gruppe -NHR gebunden ist und R Alkanoyl von 2 bis 5 C-Atomen, wie Propionyl und Acetyl, oder Benzoyl oder 2,4-Di-(cyanoamino)-1,3,5-triazin-6-yl oder eine Gruppe der allgemeinen Formel (4a) oder (4b) 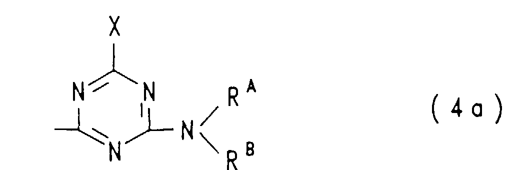 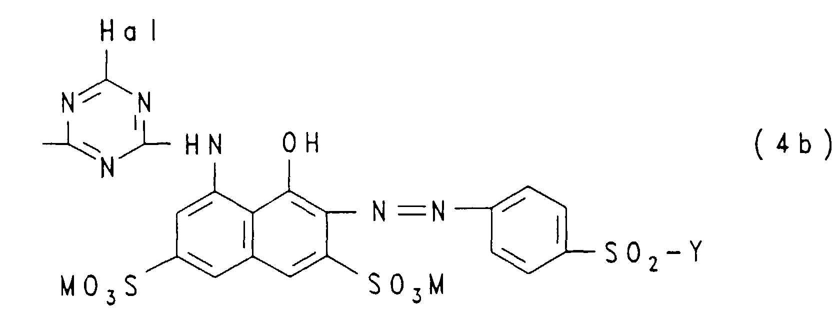 ist, in welchen M und Y eine der obengenannten Bedeutungen haben, X für Chlor, Fluor oder Cyanoamino steht, Hal Chlor oder Fluor ist, R^{A} Wasserstoff oder Alkyl von 1 bis 4 C-Atomen, wie Methyl oder Ethyl, ist, das durch Phenyl oder durch 1 oder 2 Substituenten aus der Gruppe Sulfo, Carboxy, Methyl, Ethyl, Methoxy und Ethoxy substituiertes Phenyl substituiert sein kann, und bevorzugt Wasserstoff ist und R^{B} Alkyl von 1 bis 4 C-Atomen, wie Methyl oder Ethyl, ist, das durch Phenyl oder durch 1 oder 2 Substituenten aus der Gruppe Sulfo, Carboxy, Methyl, Ethyl, Methoxy und Ethoxy substituiertes Phenyl substituiert sein kann, oder Monosulfo-phenyl oder Disulfo-phenyl, bevorzugt 3-Sulfo-phenyl, ist oder R^{A} und R^{B} zusammen mit dem N-Atom einen gesättigten heterocyclischen Rest mit einem Alkylen von 4 bis 7 C-Atomen, bevorzugt von 5 C-Atomen, oder mit zwei Alkylenresten von 1 bis 4 C-Atomen und einer weiteren Heterogruppe, wie -O- oder -NH- , bilden, wie beispielsweise den N-Piperidino-, N-Piperazino- oder bevorzugt N-Morpholino-Rest.

Eine Sulfogruppe ist eine Gruppe der allgemeinen Formel -SO₃M , eine Carboxygruppe ist eine Gruppe der allgemeinen Formel -COOM , eine Phosphatogruppe ist eine Gruppe der allgemeinen Formel -OPO₃M₂ , eine Sulfatogruppe ist eine Gruppe der allgemeinen Formel -OSO₃M und eine Thiosulfatogruppe ist eine Gruppe der allgemeinen Formel -S-SO₃M .

Die Gruppen -SO₂-Y stehen in den Formeln (1), (2) und (3) bevorzugt in meta-Stellung und insbesondere bevorzugt in para-Stellung zur Azogruppe an den Benzolkern gebunden.

Die Farbstoffe der allgemeinen Formel (1) sind in der Literatur beschrieben; beispielsweise sind sie aus der U.S.-Patentschrift 2 657 205, aus der japanischen Patentanmeldungs-Veröffentlichung Sho-58-160 362 und aus der U.S.-Patentschrift 4 257 770 und der dort genannten Literatur bekannt. Die Monoazofarbstoffe der allgemeinen Formel (2) sind teilweise beschrieben, beispielsweise in der europäischen Patentanmeldung-Veröffentlichung Nr. 0 040 806, oder können analog den dort gemachten Angaben synthetisiert werden. Die Farbstoffe der Formeln (3) sind ebenfalls literaturbekannt, beispielsweise aus den europäischen Patentanmeldungs-Veröffentlichungen Nrs. 0 032 187 und 0 542 214 sowie aus der deutschen Patentschrift Nr. 2 748 965, oder können analog bekannten Verfahrensweisen der Kupplung der entsprechenden Diazo- und Kupplungskomponenten hergestellt werden.

Reste von Diazokomponenten in den allgemeinen Formeln (1), (2) und (3) sind beispielsweise 3-(β-Sulfatoethylsulfonyl)-phenyl und 4-(β-Sulfatoethylsulfonyl)phenyl sowie deren Vinylsulfonyl-, β-Chlorethylsulfonyl- und β-Thiosulfatoethylsulfonyl- und β-Phosphatoethylsulfonyl-Derivate; hiervon bevorzugt sind 4-(β-Sulfatoethylsulfonyl)-phenyl und 4-Vinylsulfonyl-phenyl.

Reste der allgemeinen Formel (5) 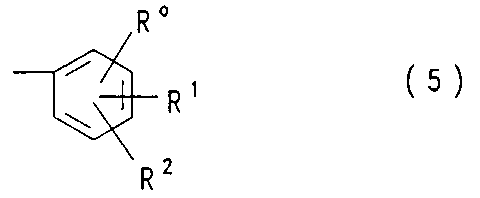 in den Farbstoffen der allgemeinen Formel (2) sind beispielsweise 2-Sulfophenyl, 3-Sulfo-phenyl, 4-Sulfo-phenyl, 2-Carboxy-phenyl, 3-Carboxy-phenyl, 4-Carboxy-phenyl, 3-Sulfo-4-methyl-phenyl, 2,5-Disulfo-phenyl, 2-Carboxy-5-sulfo-phenyl, 3,5-Dicarboxy-phenyl, 2,3-Dicarboxy-phenyl, 2,5-Dicarboxyphenyl, 2-Sulfo-4-methoxy-phenyl und 4-Sulfo-2,5-dimethoxy-phenyl.

Reste R in den Farbstoffen der allgemeinen Formel (3c) sind außer den bereits genannten Alkanoyl- und Benzoyl- sowie 2,4-Di-(cyanoamino)-1,3,5-triazin-6-yl-Gruppen beispielsweise 2-Chlor-4-(3'-sulfophenylamino)-1,3,5-triazin-6-yl, 2-Fluor-4-(3'-sulfophenyiamino)-1,3,5-triazin-6-yl, 2-Cyanoamino-4-(3'-sulfophenylamino)-1,3,5-triazin-6-yl, 2-Cyanoamino-4-(4'-sulfophenylamino)-1,3,5-triazin-6-yl, 2-Chlor-4-(4'-sulfophenylamino)-1,3,5-triazin-6-yl, 2-Fluor-4-(4'-sulfophenylamino)-1,3,5-triazin-6-yl, 2-Fluor-4-(2'-sulfophenylamino)-1,3,5-triazin-6-yl, 2-Chlor-4-(2'-sulfophenylamino)-1,3,5-triazin-6-yl, 2-Chlor-4-{4'-[7'-(4"-β-sulfatoethylsulfonyl)-azo-3',6'-disulfo-8'-hydroxy-naphth-1'-yl)-amino-1,3,5-triazin-6-yl, 2-Fluor-4-{4'-[7'-(4"-β-sulfatoethylsulfonyl)-azo-3',6'-disulfo-8'-hydroxy-naphth-1'-yl}-amino-1,3,5-triazin-6-yl und 2-Fluor-4-morpholino-1,3,5-triazin-6-yl.

Die Farbstoffe der Formel (1) und ebenso die Farbstoffe der Formel (2) können, insbesondere bei gleichem Chromophor, innerhalb der Bedeutung von Y unterschiedliche faserreaktive Gruppen -SO₂-Y besitzen. Insbesondere können die Farbstoffmischungen Farbstoffe gleichen Chromophors entsprechend der allgemeinen Formel (1) und/oder Farbstoffe gleichen Chromophors entsprechend der allgemeinen Formel (2) enthalten, in denen die faserreaktiven Gruppen -SO₂-Y zum einen Vinylsulfonylgruppen und zum anderen β-Chlorethylsulfonyl- oder β-Thiosulfatoethylsulfonyl- oder bevorzugt β-Sulfatoethylsulfonyl-Gruppen sind. Ist in den Farbstoffgemischen solch eine Farbstoffkomponente in Form eines Vinylsulfonyl-Farbstoffes vorhanden, so liegt der Farbstoffanteil dieses Vinylsulfonyl-Farbstoffes zu dem entsprechenden β-Chlor- oder β-Thiosulfato- oder β-Sulfatoethylsulfonyl-Farbstoff bei bis zu etwa 30 Mol-%, bezogen auf den Farbstoffchromophor, vor.

Hierbei sind solche Farbstoffmischungen bevorzugt, bei welchen der Anteil an den Vinylsulfonyl-Farbstoffen zu den β-Sulfatoethylsulfonyl-Farbstoffen im Molverhältnis zwischen 2:98 und 30:70 liegt.

Die erfindungsgemäßen Farbstoffmischungen können als Präparation in fester oder in flüssiger (gelöster) Form vorliegen. Sie enthalten im allgemeinen die bei wasserlöslichen und insbesondere faserreaktiven Farbstoffen üblichen Elektrolytsalze, wie Natriumchlorid, Kaliumchlorid und Natriumsulfat, und können desweiteren die in Handelsfarbstoffen üblichen Hilfsmittel enthalten, wie Puffersubstanzen, die einen pH-Wert in wäßriger Lösung zwischen 3 und 7 einzustellen vermögen, wie Natriumacetat, Natriumborat, Natriumhydrogencarbonat, Natriumdihydrogenphosphat und Dinatriumhydrogenphosphat, bei Vorliegen in fester Form geringe Mengen an Sikkativen und in flüssiger, wäßriger Lösung (einschließlich des Gehaltes von Verdickungsmitteln, wie sie bei Druckpasten üblich sind) Substanzen, die die Haltbarkeit dieser Präparationen gewährleisten, wie beispielsweise schimmelverhütende Mittel.

Im allgemeinen liegen die erfindungsgemäßen Farbstoffmischungen als Farbstoffpulver mit einem Gehalt von 10 bis 80 Gew.-%, bezogen auf das Farbstoffpulver bzw. die Präparation, an einem Elektrolytsalz, das auch als Stellmittel bezeichnet wird, vor. Diese Farbstoffpulver können zudem die erwähnten Puffersubstanzen in einer Gesamtmenge von bis zu 5 %, bezogen auf das Farbstoffpulver, enthalten. Sofern die erfindungsgemäßen Farbstoffmischungen in wäßriger Lösung vorliegen, so beträgt der Gesamtfarbstoffgehalt in diesen wäßrigen Lösungen bis zu etwa 50 Gew.-%, wie beispielsweise zwischen 5 und 55 Gew.-%., wobei der Elektrolytsalzgehalt in diesen wäßrigen Lösungen bevorzugt unterhalb 10 Gew.-%, bezogen auf die wäßrige Lösung, beträgt; die wäßrigen Lösungen (Flüssigpräparationen) können die erwähnten Puffersubstanzen in der Regel in einer Menge von bis zu 5 Gew.-%, bevorzugt bis zu 2 Gew.-%, enthalten.

Die erfindungsgemäßen Farbstoffmischungen können in üblicher Weise hergestellt werden, so durch mechanisches Mischen der einzelnen Farbstoffe in den erforderlichen Anteilen oder durch Synthese mittels der üblichen Diazotierungs- und Kupplungsreaktionen unter Verwendung von entsprechenden Mischungen der Diazo- und Kupplungskomponenten in einer dem Fachmann geläufigen Weise und den hierzu erforderlichen Mengenanteilen. So kann man beispielsweise in der Weise vorgehen, daß man 1-Amino-8-naphthol-3,6-disulfonsäure mit einem entsprechenden Überschuß eines Diazoniumsalzes des Amins der allgemeinen Formel (6) 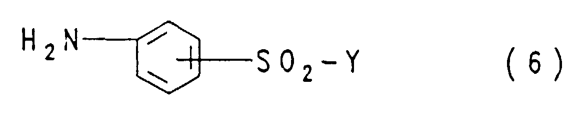 mit Y der obengenannten Bedeutung bei einem pH-Wert zwischen 0,3 und 2,5 und einer Temperatur zwischen 0 und 20°C zur obengenannten Monoazoverbindung der allgemeinen Formel (3b) umsetzt, danach den pH-Wert auf 4 bis 7 erhöht, zu dem Ansatz eine Verbindung der allgemeinen Formel (7) 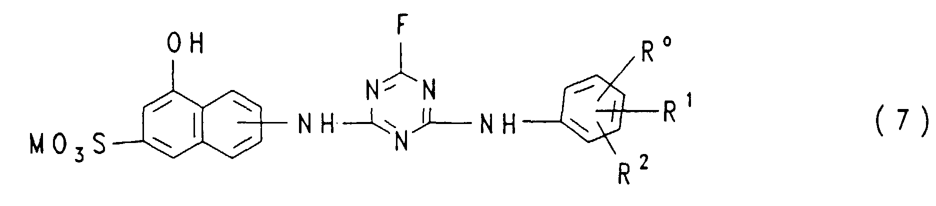 mit M, R⁰, R¹ und R² der obengenannten Bedeutung zugibt und die Kupplungsreaktion zu den Azofarbstoffen der allgemeinen Formeln (1) und (2) bei einem pH-Wert zwischen 4 und 7, bevorzugt zwischen 5 und 6, und einer Temperatur zwischen 0 und 20°C zu Ende führt. Man kann die erfindungsgemäße Farbstoffmischung auf chemischem Wege auch so herstellen, daß man Ausgangsverbindungen der allgemeinen Formeln (3b) und (7) miteinander mechanisch mischt, in Wasser löst (oder wäßrige Lösungen dieser Komponenten miteinander vermischt), die entsprechende Menge an dem Diazoniumsalz des Amins der allgemeinen Formel (6) hinzugibt und die Kupplungsreaktionen bei einem pH-Wert zwischen 3 und 7, bevorzugt zwischen 4 und 6, und einer Temperatur zwischen 0 und 20° C durchführt.

Die so erhaltene Farbstoffmischung kann aus der Lösung in üblicher Weise isoliert werden, so beispielsweise durch Aussalzen mit einem Elektrolytsalz, wie Natriumchlorid, Kaliumchlorid oder Lithiumchlorid, oder durch Sprühtrocknung.

Werden die erfindungsgemäßen Farbstoffmischungen durch mechanisches Mischen der Einzelfarbstoffe hergestellt, so werden beim Mischen eventuell erforderliche Stellmittel, Entstaubungsmittel oder weitere Hilfsmittel, die in der Färbereitechnik oder in den hierzu verwendeten Farbstoffpräparationen üblich sind, zugegeben.

Von den Verbindungen der allgemeinen Formel (2) sind Verbindungen der allgemeinen Formel (2A) 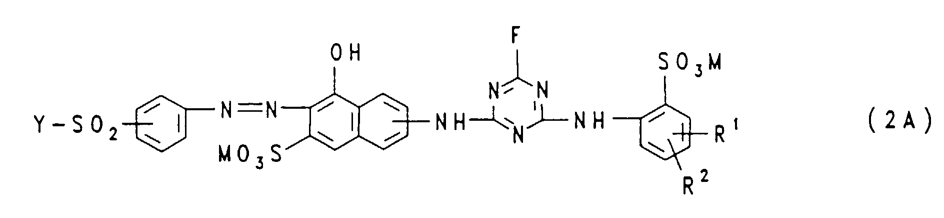 in welcher M, Y, R¹ und R² eine der obengenannten Bedeutungen besitzt und der Fluortriazinylamino-Rest in 2- oder 3-Stellung an den 8-Hydroxy-naphthalin-Rest gebunden ist, noch nicht beschrieben und somit neu. Die vorliegende Erfindung betrifft deshalb auch die Monoazoverbindungen der allgemeinen Formel (2A), ein Verfahren zu deren Herstellung und deren Verwendung als Farbstoffe.

Die Verbindungen der allgemeinen Formel (2A) lassen sich erfindungsgemäß herstellen, indem man eine Verbindung der allgemeinen Formel (6) in üblicher und bekannter Weise diazotiert (beispielsweise in salzsaurer Lösung mittels einem Alkalinitrit bei einem pH-Wert unterhalb 2 und einer Temperatur zwischen -5° C und + 10°C) und mit einer Verbindung der allgemeinen Formel (7A) 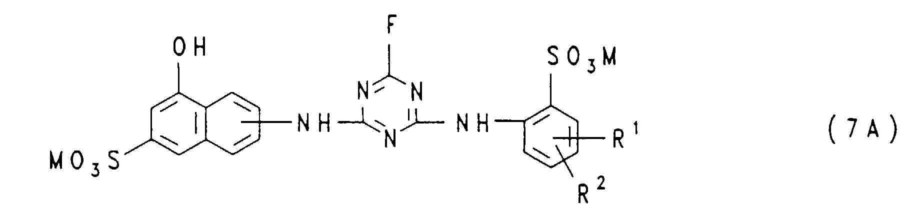 in welcher M, R¹ und R² eine der obengenannten Bedeutungen haben und der Fluortriazinylamino-Rest in 2- oder 3-Stellung an den 8-Hydroxy-naphthalin-Rest gebunden ist, bei einem pH-Wert zwischen 3 und 7, bevorzugt zwischen 4 und 6, und bei einer Temperatur zwischen 0 und 20°C kuppelt, oder daß man eine Verbindung der allgemeinen Formel (8) 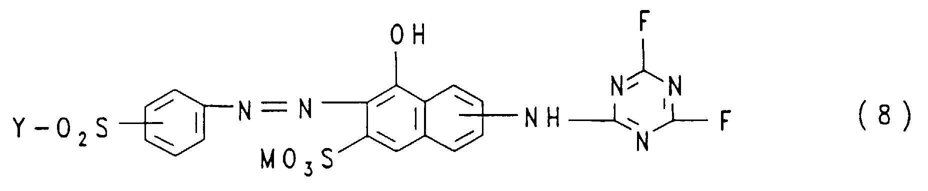 in welcher Y und M die obengenannten Bedeutungen haben und der Difluor-triazinylamino-Rest in 2- oder 3-Stellung an den 8-Hydroxy-6-sulfo-naphthalin-Rest gebunden ist, mit einer Verbindung der allgemeinen Formel (9) 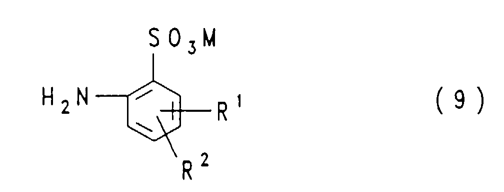 mit M, R¹ und R² der oben angegebenen Bedeutung bei einem pH-Wert zwischen 2 und 7, bevorzugt zwischen 3 und 6, und bei einer Temperatur zwischen 5 und 20°C, bevorzugt zwischen 5 und 10°C, umsetzt, oder daß man eine Verbindung der allgemeinen Formel (10) 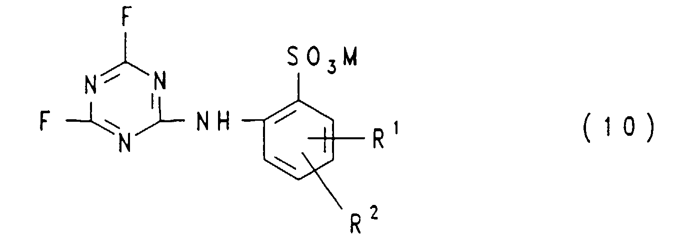 mit M, R¹ und R² der oben angegebenen Bedeutung bei einem pH-Wert zwischen 4 und 7, bevorzugt zwischen 5 und 6, und bei einer Temperatur zwischen -5°C und +20°C, bevorzugt zwischen 0 und 10°C, mit einer Verbindung der allgemeinen Formel (11) 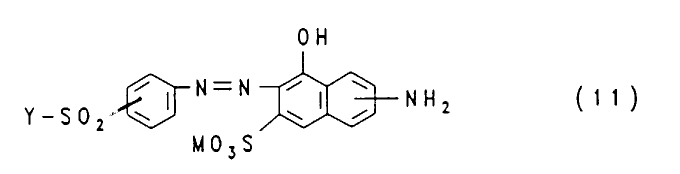 in welcher M und Y die oben genannten Bedeutungen haben und die freie Aminogruppe in 2- oder 3-Stellung an den 8-Hydroxy-6-sulfo-naphthalin-Rest gebunden ist, umsetzt.

Die Ausgangsverbindungen der allgemeinen Formel (7A) lassen sich herstellen, indem man analog der in der deutschen Offenlegungsschrift 2 746 109 beschriebenen Verfahrensweise Trifluortriazin mit einer Aminoverbindung der allgemeinen Formel (9) umsetzt und die erhaltenen Difluor-triazinylamino-Verbindung der Formel (10) mit 2- oder 3-Amino-6-sulfo-8-naphthol bei einer Temperatur zwischen 0 und 20° C, bevorzugt zwischen 5 und 10°C, und bei einem pH-Wert zwischen 2 und 7, bevorzugt zwischen 3 und 6,5, umsetzt. Die Ausgangsverbindungen der allgemeinen Formel (8) lassen sich herstellen, indem man die durch Kupplungsreaktion von 2- oder 3-Amino-6-sulfo-8-naphthol mit dem Diazoniumsalz des Amins der allgemeinen Formel (6) erhaltene Azoverbindung mit Cyanurfluorid (Trifluortriazin) bei einer Temperatur zwischen 0 und 5°C und einem pH-Wert zwischen 2 und 7, bevorzugt zwischen 3 und 6, umsetzt.

Die erfindungsgemäßen Monoazoverbindungen der allgemeinen Formel (2A) besitzen sehr gute Farbstoffeigenschaften. Sie liefern nach den in der Technik für faserreaktive Farbstoffe üblichen Applikations- und Fixierverfahren auf hydroxy- und/oder carbonamidgruppenhaltigen Fasermaterialien farbstarke, echte Färbungen und Drucke mit orangen bis braunen Farbtönen.

Hydroxygruppenhaltige Fasermaterialien sind solche natürlichen oder synthetischen Ursprungs, wie beispielsweise Cellulosefasermaterialien, auch in Form von Papier, oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Fasermaterialien sind beispielsweise Fasern aus synthetischen und natürlichen Polyamiden oder aus Polyurethanen, wie beispielsweise Wolle und andere Tierhaare, Seide, Leder, Polyamid-6,6, Polyamid-6, Polyamid-11 und Polyamid-4.

Die erfindungsgemäßen Farbstoffmischungen aus den Farbstoffen der Formeln (1) und (2) liefern nach den in der Technik für faserreaktive Farbstoffe zahlreich beschriebenen Anwendungs- und Fixierverfahren auf hydroxy- und/oder carbonamidgruppenhaltigen Fasermaterialien tiefschwarze Färbungen mit gutem Farbaufbau und guter Auswaschbarkeit nicht fixierter Farbstoffanteile.

Gegenstand der vorliegenden Erfindung ist somit auch die Verwendung der erfindungsgemäßen Monoazoverbindungen der Formel (2A) und der erfindungsgemäßen Farbstoffmischungen oder der Farbstoffe der Formeln (1) und (2) gemeinsam zum Färben (einschließlich Bedrucken) von hydroxy- und/oder carbonamidgruppenhaltigen Fasermaterialien bzw. Verfahren zum Färben solcher Fasermaterialien unter Verwendung eines erfindungsgemäßen Farbstoffes der Formel (2A) oder einer erfindungsgemäßen Farbstoffmischung oder der Farbstoffe der Formeln (1) und (2) gemeinsam, indem man diese in gelöster Form auf das Substrat appliziert und die Farbstoffe durch Einwirkung eines alkalisch wirkenden Agens oder durch Hitze oder durch beide Maßnahmen auf der Faser fixiert.

Die Anwendung der erfindungsgemäßen Farbstoffe und Farbstoffmischungen erfolgt nach allgemein bekannten Verfahren zum Färben und Bedrucken von Fasermaterialien gemäß den bekannten Anwendungstechniken für faserreaktive Farbstoffe. Da die Farbstoffe der erfindungsgemäßen Farbstoffmischungen zueinander ein sehr gutes Kombinationsverhalten zeigen, können die erfindungsgemäßen Farbstoffmischungen auch mit Vorteil in den Ausziehfärbeverfahren eingesetzt werden. Demgemäß erhält man mit ihnen beispielsweise auf Cellulosefasern nach den Ausziehverfahren aus langer Flotte bei Temperaturen zwischen 40 und 105°C, gegebenenfalls bei Temperaturen bis zu 130°C unter Druck, und gegebenenfalls in Gegenwart von üblichen Färbereihilfsmitteln unter Verwendung von säurebindenden Mitteln und gegebenenfalls neutralen Salzen, wie Natriumchlorid oder Natriumsulfat, Färbungen in sehr guten Farbausbeuten und mit ausgezeichnetem Farbaufbau und gleicher Nuance. Man kann dabei so vorgehen, daß man das Material in das warme Bad einbringt und dieses allmählich auf die gewünschte Färbetemperatur erwärmt und den Färbeprozeß bei dieser Temperatur zu Ende führt. Die das Ausziehen der Farbstoffe beschleunigenden Neutralsalze können dem Bade gewünschtenfalls auch erst nach Erreichen der eigentlichen Färbetemperatur zugesetzt werden.

Ebenfalls erhält man nach den üblichen Druckverfahren für Cellulosefasern - die entweder einphasig durchgeführt werden können, beispielsweise durch Bedrucken mit einer Natriumbicarbonat oder ein anderes säurebindendes Mittel und das Farbmittel enthaltenden Druckpaste und durch anschließendes Dämpfen bei 100 bis 103°C, oder die zweiphasig, beispielsweise durch Bedrucken mit neutraler oder schwach saurer, das Farbmittel enthaltender Druckpaste und anschließendes Fixieren entweder durch Hindurchführen der bedruckten Ware durch ein heißes elektrolythaltiges alkalisches Bad oder durch Überklotzen mit einer alkalischen elektrolythaltigen Klotzflotte mit anschließendem Verweilen dieses behandelten Materials oder anschließendem Dämpfen oder anschließender Behandlung mit Trockenhitze, durchgeführt werden können, - farbstarke Drucke mit gutem Stand der Konturen und einem klaren Weißfond. Der Ausfall der Drucke ist von wechselnden Fixierbedingungen nur wenig abhängig. Sowohl in der Färberei als auch in der Druckerei sind die mit den erfindungsgemäßen Farbstoffmischungen erhaltenen Fixiergrade sehr hoch. Bei der Fixierung mittels Trockenhitze nach den üblichen Thermofixierverfahren verwendet man Heißluft von 120 bis 200°C. Neben dem üblichen Wasserdampf von 101 bis 103°C kann auch überhitzter Dampf und Druckdampf von Temperaturen bis zu 160°C eingesetzt werden.

Die säurebindenden und die Fixierung der Farbstoffe auf den Cellulosefasern bewirkenden Mittel sind beispielsweise wasserlösliche basische Salze der Alkalimetalle und der Erdalkalimetalle von anorganischen oder organischen Säuren, ebenso Verbindungen, die in der Hitze Alkali freisetzen. Insbesondere sind die Alkalimetallhydroxide und Alkalimetallsalze von schwachen bis mittelstarken anorganischen oder organischen Säuren zu nennen, wobei von den Alkaliverbindungen vorzugsweise die Natrium- und Kaliumverbindungen gemeint sind. Solche säurebindenden Mittel sind beispielsweise Natriumhydroxid, Kaliumhydroxid, Natriumcarbonat, Natriumbicarbonat, Kaliumcarbonat, Natriumformiat, Natriumdihydrogenphosphat und Dinatriumhydrogenphosphat.

In gleicher Weise, wie oben für die Farbstoffmischungen beschrieben, können die erfindungsgemäßen Verbindungen der Formel (2A) in die Färbe- und Druckprozesse eingesetzt werden.

Durch die Behandlung der Farbstoffe mit den säurebindenden Mitteln, gegebenenfalls unter Wärmeeinwirkung, werden die Farbstoffe chemisch an die Cellulosefaser gebunden; insbesondere die Cellulosefärbungen zeigen nach der üblichen Nachbehandlung durch Spülen zur Entfernung von nicht fixierten Farbstoffanteilen ausgezeichnete Naßechtheiten, zumal sich nicht fixierte Farbstoffanteile leicht wegen ihrer guten Kaltwasserlöslichkeit auswaschen lassen.

Die Färbungen auf Polyurethan- und Polyamidfasern werden üblicherweise aus saurem Milieu ausgeführt. So kann man beispielsweise dem Färbebad Essigsäure und/oder Ammoniumsulfat oder Essigsäure und Ammoniumacetat oder Natriumacetat zufügen, um den gewünschten pH-Wert zu erhalten. Zur Erreichung einer brauchbaren Egalität der Färbung empfiehlt sich ein Zusatz an üblichen Egalisierhilfsmitteln, wie beispielsweise auf Basis eines Umsetzungsproduktes von Cyanurchlorid mit der dreifach molaren Menge einer Aminobenzolsulfonsäure oder Aminonaphthalinsulfonsäure oder auf Basis eines Umsetzungsproduktes von beispielsweise Stearylamin mit Ethylenoxid. In der Regel wird das zu färbende Material bei einer Temperatur von etwa 40°C in das Bad eingebracht, dort einige Zeit darin bewegt, das Färbebad dann auf den gewünschten schwach sauren, vorzugsweise schwach essigsauren pH-Wert nachgestellt und die eigentliche Färbung bei einer Temperatur zwischen 60 und 98°C durchgeführt. Die Färbungen können aber auch bei Siedetemperatur oder bei Temperaturen bis zu 120°C (unter Druck) ausgeführt werden.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile stehen zu Volumenteilen im Verhältnis von Kilogramm zu Liter.

In den Beispielen sind die Formeln der Farbstoffe in Form der freien Säure angegeben; die Mengenteile beziehen sich auf die saure Form. In der Regel werden die Farbstoffe jedoch in der für wasserlösliche Farbstoffe üblicherweise vorliegenden Form als elektrolytsalzhaltiges (beispielsweise natriumchlorid- und natriumsulfathaltiges) Alkalimetallsalz-Pulver eingesetzt.

### Beispiel 1

35,4 Teile Anilin-2-sulfonsäure werden gemäß den Angaben der deutschen Offenlegungsschrift Nr. 2 746 109 mit 29,8 Teilen 2,4,6-Trifluor-1,3,5-triazin umgesetzt. Danach werden zu dem Ansatz unter Rühren 47 Teile 3-Amino-8-naphthol-6-sulfonsäure hinzugegeben, ein pH-Wert von 6 eingestellt und die Umsetzung unter Einhaltung dieses pH-Wertes mittels einer wäßrigen Lithiumhydroxydlösung bei einer Temperatur von 5 bis 10°C während etwa drei Stunden zu Ende geführt.
Die hergestellte Verbindung wird durch Aussalzen mittels Natriumchlorid aus der Syntheselösung ausgefällt und abfiltriert.

50,7 Teile der so hergestellten und als Kupplungskomponente dienenden Verbindung 3-[2'-Fluor-4'-(2''-sulfo-phenyl)-amino-1',3',5'-triazin-6'-yl]-amino-8-naphthol-6-sulfonsäure werden in 800 Vol.-teilen Eiswasser suspendiert, und die Suspension wird mit einer salzsauren wäßrigen Suspension des auf üblichem Wege hergestellten Diazoniumsalzes aus 28,1 Teilen 4-(β-Sulfatoethylsulfonyl)-anilin innerhalb von 30 Minuten unter Einhaltung eines pH-Wertes zwischen 5 und 6 und einer Temperatur zwischen 0 und 15 °C unter Rühren versetzt (die Einhaltung des pH-Bereiches kann durch laufende Zugabe einer wäßrigen Natriumcarbonatlösung erfolgen). Man rührt dann etwa eine Stunde nach, stellt einen pH-Wert von 7 ein, setzt gegebenenfalls einige Teile Natriumdihydrogenphosphat und Dinatriumhydrogenphosphat als Phosphatpuffer hinzu und isoliert die hergestellte erfindungsgemäße Monoazoverbindung in üblicher Weise durch Sprühtrocknung oder durch Aussalzen mit Natriumchlorid als Alkalimetallsalz. Sie besitzt, in Form der freien Säure geschrieben, die Formel 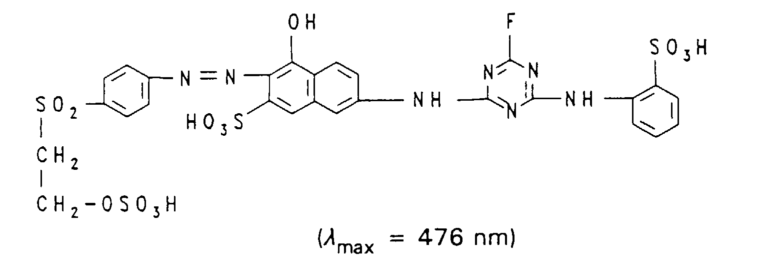 zeigt sehr gute faserreaktive Farbstoffeigenschaften und liefert auf den in der Beschreibung genannten Materialien, wie insbesondere Cellulosefasermaterialien, beispielsweise Baumwolle, Färbungen und Drucke in kräftigen, goldorangen Farbtönen.

### Beispiele 2 bis 9

In den nachfolgenden Tabellenbeispielen sind weitere erfindungsgemäße Monoazoverbindungen entsprechend der allgemeinen Formel (A) 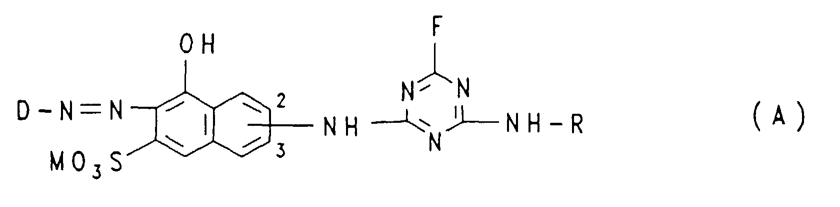 anhand ihrer Komponenten beschrieben. Sie lassen sich in erfindungsgemäßer Weise aus den aus der allgemeinen Formel (A) ersichtlichen Ausgangsverbindungen (der Diazokomponente D-NH₂ , 2- oder 3-Amino-6-sulfo-8-naphthol, Cyanurfluorid und dem Anilin der Formel H₂N-R) herstellen. Sie besitzen sehr gute faserreaktive Farbstoffeigenschaften und färben die in der Beschreibung genannten Materialien, wie insbesondere Cellulosefasermaterialien, in dem in dem jeweiligen Tabellenbeispiel (hier für Baumwolle) angegebenen Farbton in hoher Farbstärke und mit guten Echtheiten.

| | Azoverbindung der Formel (A) | | | |
|---|---|---|---|---|
| Bsp. | Rest D | Gruppe -NH- | Rest R | Farbton |
| 2 | 4-(β-Sulfatoethylsulfonyl)-phenyl | in 3-Stellung | 4-Methyl-2-sulfophenyl | orange |
| 3 | dito | in 3-Stellung | 2,5-Disulfo-phenyl | orange |
| 4 | dito | in 2-Stellung | 2-Sulfo-phenyl | orangebraun |
| 5 | dito | in 2-Stellung | 2,5-Disulfo-phenyl | orangebraun |
| 6 | dito | in 2-Stellung | 4-Methyl-2-sulfo-phenyl | orangebraun |
| 7 | 4-(Vinylsulfonyl)-phenyl | in 2-Stellung | 2,5-Disulfo-phenyl | orangebraun |
| 8 | 4-(β-Chlorethylsulfonyl)-phenyl | in 2-Stellung | dito | orangebraun |
| 9 | 3-(β-Sulfatoethylsulfonyl)-phenyl | in 2-Stellung | dito | orangebraun |

### Beispiel A

40 Teile eines Disazofarbstoffes entsprechend der allgemeinen Formel (1), in welcher beide Gruppen -SO₂-Y den Rest β-Sulfatoethylsulfonyl darstellen, die beide in para-Stellung zur Azogruppe an den jeweiligen Benzolkern gebunden sind, und 13 Teile des Farbstoffes der Formel 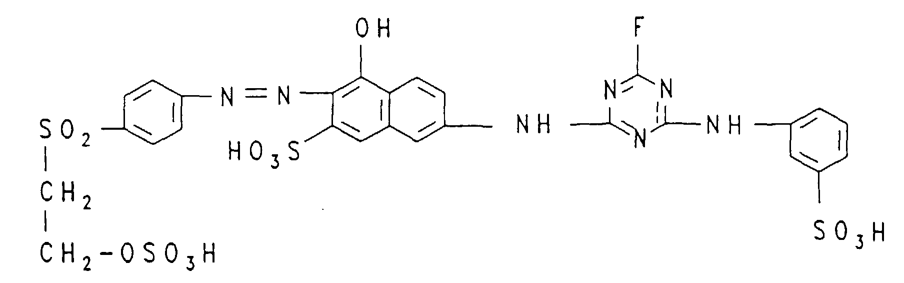 werden miteinander homogen vermahlen. Die erfindungsgemäße Farbstoffmischung liefert beispielsweise auf Baumwolle nach den in der Technik für faserreaktive Farbstoffe üblichen Anwendungsverfahren kräftige, tiefschwarze Färbungen und Drucke mit guten Echtheiten, von denen insbesondere die guten Waschechtheiten oder die gute Auswaschbarkeit nicht auf dem Fasermaterial fixierter Farbstoffanteile aus der Färbung hervorgehoben werden können.

### Beispiel B

40 Teile eines Disazofarbstoffes entsprechend der allgemeinen Formel (1), in welcher beide Gruppen -SO₂-Y den Rest β-Sulfatoethylsulfonyl darstellen, die beide in para-Stellung zur Azogruppe an den jeweiligen Benzolkern gebunden sind, und 13 Teile des Monoazofarbstoffes von Beispiel 1 werden miteinander homogen vermahlen. Die erfindungsgemäße Farbstoffmischung liefert beispielsweise auf Baumwolle nach den in der Technik für faserreaktive Farbstoffe üblichen Anwendungsverfahren kräftige, tiefschwarze Färbungen und Drucke mit guten Echtheiten, von denen insbesondere die guten Waschechtheiten oder die gute Auswaschbarkeit nicht auf dem Fasermaterial fixierter Farbstoffanteile aus der Färbung hervorgehoben werden können.

### Beispiel C

Man diazotiert 37,8 Teile 4-(β-Sulfatoethylsulfonyl)-anilin in üblicher Weise und rührt diese salzsaure Diazoniumsalzsuspension in eine Suspension aus 32 Teilen 1-Amino-8-naphthol-3,6-disulfonsäure in 600 Teilen Eiswasser ein. Man rührt den Ansatz noch vier Stunden bei 10°C unter Einhaltung eines pH-Wertes zwischen 1 und 1,5 mittels Natriumhydrogencarbonat weiter und gibt danach 17,8 Teile der analog der Verfahrensweise des Beispieles 1 herstellbaren Verbindung 3-[2'-Fluor-4'-(3"-sulfo-phenyl)-amino-1',3',5'-triazin-6'-yl]-amino-6-sulfo-8-naphthol hinzu, stellt einen pH-Wert zwischen 5 und 6 ein und rührt den Ansatz noch etwa drei Stunden unter Einhaltung dieses pH-Bereiches bei einer Temperatur von etwa 5°C weiter. Danach stellt man den Ansatz auf einen pH-Wert von 7, gibt gegebenenfalls einige Teile eines Phosphatpuffers hinzu und isoliert die erfindungsgemäße Farbstoffmischung durch Sprühtrocknung.

Sie enthält 88 Teile des Disazofarbstoffes 2,7-Di-[4'-(β-sulfatoethylsulfonyl)-phenyl-azo]-3,6-disulfo-1-amino-8-hydroxy-naphthalin und 28 Teile des Monoazofarbstoffes 7-[4'-(β-Sulfatoethylsulfonyl)-phenyl-azo]-6-sulfo-3-amino-[2"-fluor-4"-(3"'-sulfo-phenyl)-amino-1",3",5"-triazin-6"-yl]-amino-8-hydroxynaphthalin. Die erfindungsgemäße Farbstoffmischung zeigt sehr gute anwendungstechnische Eigenschaften und färbt beispielsweise Baumwolle nach den in der Technik für faserreaktive Farbstoffe üblichen Applikations- und Fixierverfahren in kräftigen, tiefschwarzen Tönen.

### Beispiel D

Man verfährt gemäß der Verfahrensweise des Beispieles C, setzt jedoch anstelle der dort verwendeten Fluor-triazinylamino-naphthol-Verbindung 25 Teile der Verbindung 3-[2'-Fluor-4'-(2"-sulfo-phenyl)-amino-1',3',5'-triazin-6'-yl]-amino-6-sulfo-8-naphthol als Kupplungskomponente ein. Man erhält eine Farbstoffmischung, die beispielsweise Baumwolle nach den in der Technik üblichen Färbe- und Druckverfahren in tiefschwarzen Tönen färbt.

### Beispiel E

Man verfährt gemäß der Verfahrensweise des Beispieles C, setzt jedoch anstelle der dort verwendeten Fluor-triazinylamino-naphthol-Verbindung 23 Teile der Verbindung 3-[2'-Fluor-4'-(2"-sulfo-4"-methyl-phenyl)-amino-1',3',5'-triazin-6'-yl]-amino-6-sulfo-8-naphthol als Kupplungskomponente ein.
Man erhält eine Farbstoffmischung, die beispielsweise Baumwolle nach den in der Technik üblichen Färbe- und Druckverfahren in tiefschwarzen Tönen färbt.

### Beispiel F

Zur Herstellung einer erfindungsgemäßen Farbstoffmischung verfährt man gemäß den Angaben des Beispieles C, setzt jedoch anstelle von 37,8 Teilen 4-(β-Sulfatoethylsulfonyl)-anilin 43 Teile dieser Verbindung in die Diazotierungsreaktion und als zusätzliche Kupplungskomponente 10 Teile der Verbindung 1-[2'-Chlor-4'-(3"-sulfo-phenyl)-amino-1',3',5'-triazin-6'-yl]-amino-3,6-disulfo-8-naphthol ein.
Die erfindungsgemäße Farbstoffmischung liefert nach den in der Technik für faserreaktive Farbstoffe üblichen Färbe- und Druckverfahren tiefschwarze Färbungen und Drucke.

### Beispiel G

Man verfährt gemäß der Verfahrensweise des Beispieles F, setzt jedoch anstelle der dort angegebenen 1-Chlortriazinylamino-3,6-disulfo-8-naphthol-Verbindung 10 Teile der Verbindung 1-(2'-Fluor-4'-morpholino-1',3',5'-triazin-6'-yl)-amino-3,6-disulfo-8-naphthol ein.
Man erhält eine Farbstoffmischung, die beispielsweise Baumwolle in tiefschwarzen Tönen färbt.

## Patentansprüche

1. Farbstoffmischung, enthaltend einen oder mehrere Disazofarbstoffe entsprechend der allgemeinen Formel (1) und einen oder mehrere Monoazofarbstoffe entsprechend der allgemeinen Formel (2) 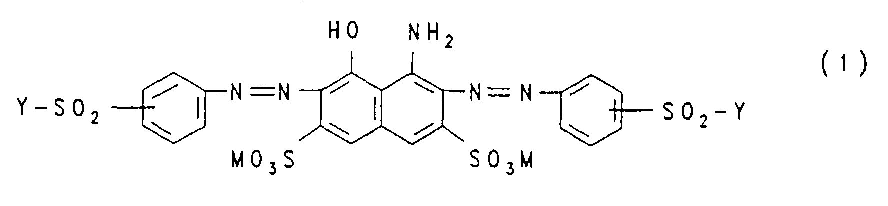 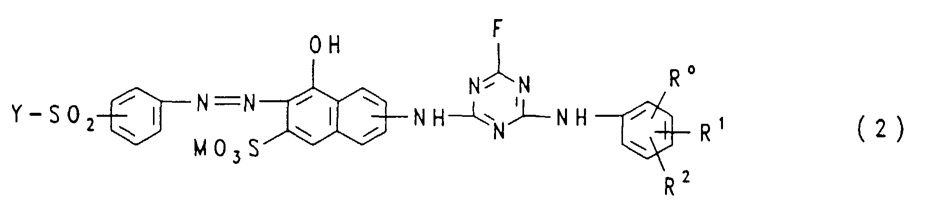 worin bedeuten:
M ist Wasserstoff oder ein Alkalimetall;
R⁰ ist Sulfo oder Carboxy;
R¹ ist Wasserstoff, Methyl, Ethyl, Methoxy, Ethoxy, Sulfo oder Carboxy;
R² ist Wasserstoff, Methyl, Ethyl, Methoxy oder Ethoxy;
Y ist jedes, unabhängig voneinander, Vinyl, β-Chlorethyl, β-Thiosulfatoethyl, β-Phosphatoethyl oder β-Sulfatoethyl;
in Formel (2) ist die Fluortriazinylamino-Gruppe in 2- oder 3-Stellung an den 8-Hydroxy-6-sulfo-naphthalin-Rest gebunden.

2. Farbstoffmischung nach Anspruch 1, dadurch gekennzeichnet, daß die Farbstoffe der allgemeinen Formel (1) und die Farbstoffe der allgemeinen Formeln (2) in einem molaren Mischungsverhältnis von 1:0,3 bis 1:1,4 in der Mischung enthalten sind.

3. Farbstoffmischung nach Anspruch 1, dadurch gekennzeichnet, daß die Farbstoffe der allgemeinen Formel (1) und die Farbstoffe der allgemeinen Formeln (2) in einem molaren Mischungsverhältnis von 1:0,5 bis 1:1 in der Mischung enthalten sind.

4. Monoazoverbindung der allgemeinen Formel (2A) 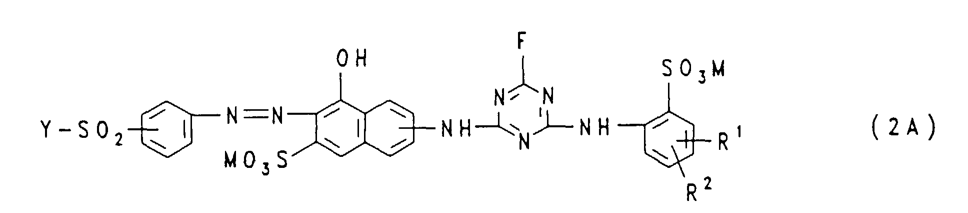 in welcher
M Wasserstoff oder ein Alkalimetall ist,
Y Vinyl, β-Chlorethyl, β-Phosphatoethyl, β-Thiosulfatoethyl oder β-Sulfatoethyl ist,
R¹ Wasserstoff, Methyl, Ethyl, Methoxy, Ethoxy, Sulfo oder Carboxy ist,
R² Wasserstoff, Methyl, Ethyl, Methoxy oder Ethoxy ist und
die Fluortriazinylamino-Gruppe in 2- oder 3-Stellung an den 8-Hydroxy-6-sulfo-naphthalinrest gebunden ist.

5. Verfahren zur Herstellung einer Monoazoverbindung der allgemeinen Formel (2A) von Anspruch 4, dadurch gekennzeichnet, daß man eine Verbindung der allgemeinen Formel (6) 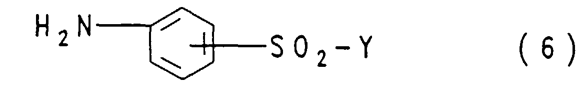 mit Y der in Anspruch 4 genannten Bedeutung diazotiert und mit einer Verbindung der allgemeinen Formel (7A) 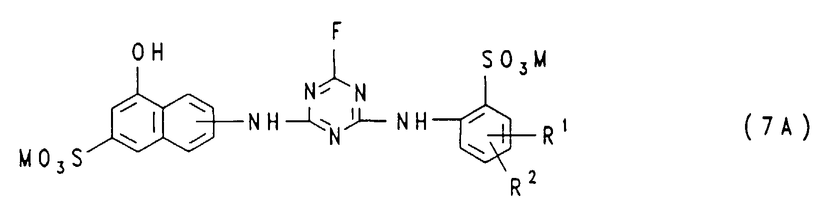 in welcher M, R¹ und R² eine der in Anspruch 4 angegebenen Bedeutungen haben und der Fluortriazinylamino-Rest in 2- oder 3-Stellung an den 8-Hydroxy-naphthalin-Rest gebunden ist, bei einem pH-Wert zwischen 3 und 7 und einer Temperatur zwischen 0 und 20°C kuppelt, oder daß man eine Verbindung der allgemeinen Formel (8) 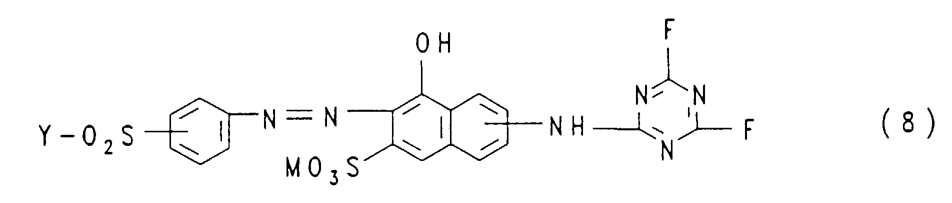 in welcher Y und M die in Anspruch 4 genannten Bedeutungen haben und der Difluor-triazinylamino-Rest in 2- oder 3-Stellung an den 8-Hydroxy-6-sulfo-naphthalin-Rest gebunden ist, mit einer Verbindung der allgemeinen Formel (9) 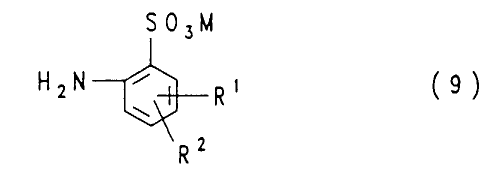 mit M, R¹ und R² der in Anspruch 4 genannten Bedeutung bei einem pH-Wert zwischen 2 und 7 und einer Temperatur zwischen 5 und 20°C umsetzt, oder daß man eine Verbindung der allgemeinen Formel (10) 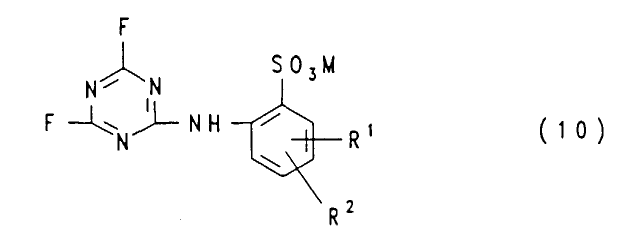 mit M, R¹ und R² der in Anspruch 4 genannten Bedeutungen bei einem pH-Wert zwischen 4 und 7 und bei einer Temperatur zwischen -5°C und + 20°C, mit einer Verbindung der allgemeinen Formel (11) 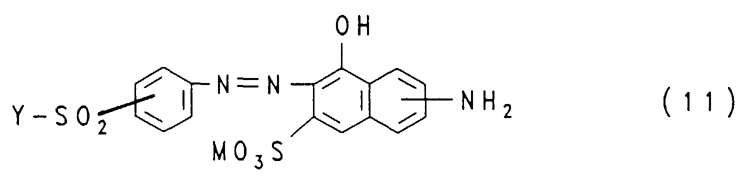 in welcher M und Y die in Anspruch 4 genannten Bedeutungen haben und die freie Aminogruppe in 2- oder 3-Stellung an den 8-Hydroxy-6-sulfo-naphthalin-Rest gebunden ist, umsetzt.

6. Verwendung einer Monoazoverbindung von Anspruch 4 zum Färben von hydroxy- und/oder carbonamidgruppenhaltigem Fasermaterial.

7. Verfahren zum Färben von hydroxy- und/oder carbonamidgruppenhaltigem Fasermaterial, bei welchem man einen Farbstoff in gelöster Form auf das Material aufbringt und den Farbstoff auf dem Material mittels Wärme oder mit Hilfe eines alkalisch wirkenden Mittels fixiert, dadurch gekennzeichnet, daß der Farbstoff eine Monoazoverbindung von Anspruch 4 ist.

8. Verwendung einer Farbstoffmischung nach mindestens einem der Ansprüche 1 bis 3 oder der Farbstoffe (1) und (2) gemeinsam zum Färben von hydroxy- und/oder carbonamidgruppenhaltigem Fasermaterial.

9. Verfahren zum Färben von hydroxy- und/oder carbonamidgruppenhaltigem Fasermaterial, bei welchem man Farbstoffe in gelöster Form auf das Material aufbringt und diese auf dem Material mittels Wärme oder mit Hilfe eines alkalisch wirkenden Mittels oder mittels beider Maßnahmen fixiert, dadurch gekennzeichnet, daß man als Farbstoffe eine Farbstoffmischung von mindestens einem der Ansprüche 1 bis 3 oder die Farbstoffe der Formeln (1) und (2) von Anspruch 1 gemeinsam appliziert und fixiert.

## Claims

1. A dye mixture comprising one or more disazo dyes of the formula (1) and one or more monoazo dyes of the formula (2) 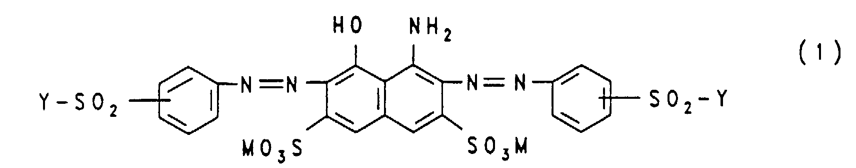 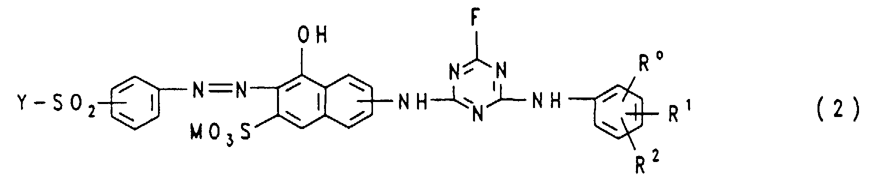 in which:
M is hydrogen or an alkali metal;
R⁰ is sulfo or carboxyl;
R¹ is hydrogen, methyl, ethyl, methoxy, ethoxy, sulfo or carboxyl;
R² is hydrogen, methyl, ethyl, methoxy or ethoxy;
Y is at each occurrence independently of the others vinyl, β-chloroethyl, β-thiosulfatoethyl, β-phosphatoethyl or β-sulfatoethyl;
in formula (2) the fluorotriazinyl group is attached in position 2 or 3 to the 8-hydroxy-6-sulfo-naphthalene radical.

2. A dye mixture as claimed in claim 1, wherein the dyes of the formula (1) and the dyes of the formulae (2) are present in the mixture in a molar ratio of from 1:0.3 to 1:1.4.

3. A dye mixture as claimed in claim 1, wherein the dyes of the formula (1) and the dyes of the formulae (2) are present in the mixture in a molar ratio of from 1:0.5 to 1:1.

4. A monoazo compound of the formula (2A) 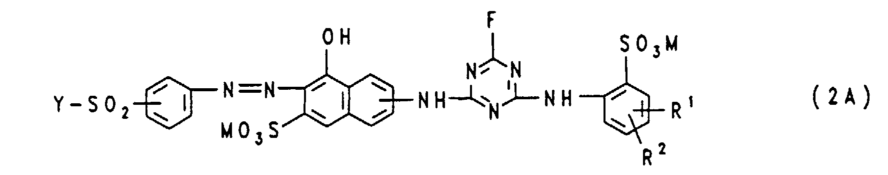 in which
M is hydrogen or an alkali metal,
Y is vinyl, β-chloroethyl, β-phosphatoethyl, (3-thiosulfatoethyl or β-sulfatoethyl,
R¹ is hydrogen, methyl, ethyl, methoxy, ethoxy, sulfo or carboxyl,
R² is hydrogen, methyl, ethyl, methoxy or ethoxy, and
the fluorotriazinylamino group is attached in position 2 or 3 to the 8-hydroxy-6-sulfonaphthalene radical.

5. A process for the preparation of a monoazo compound of the formula (2A) of claim 4, which comprises diazotizing a compound of the formula (6) 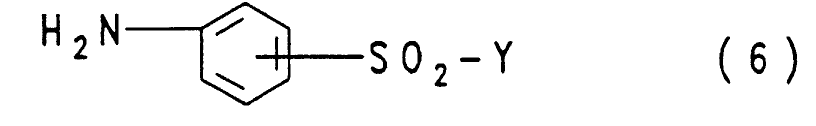 where Y is as defined in claim 4 and coupling the diazonium salt with a compound of the formula (7A) 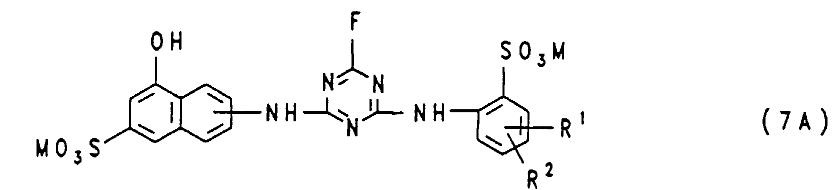 in which M, R¹ and R² are as defined in claim 4 and the fluorotriazinylamino radical is attached in position 2 or 3 to the 8-hydroxynaphthalene radical at a pH of between 3 and 7 and at a temperature of between 0 and 20°C, or comprises reacting a compound of the formula (8) 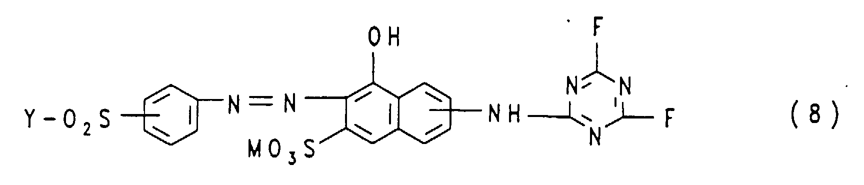 in which Y and M are as defined in claim 4 and the difluorotriazinylamino radical is attached in position 2 or 3 to the 8-hydroxy-6-sulfonaphthalene radical with a compound of the formula (9) 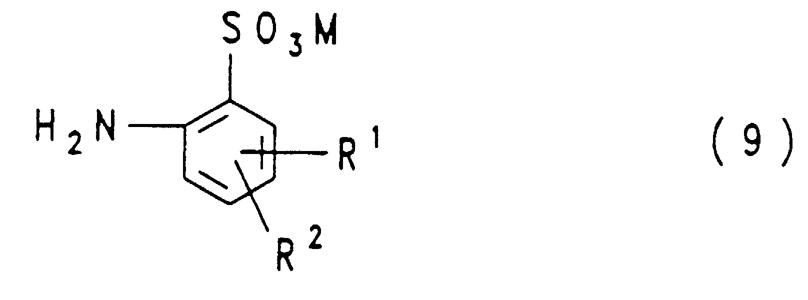 where M, R¹ and R² are as defined in claim 4 at a pH of between 2 and 7 and at a temperature of between 5 and 20°C, or comprises reacting a compound of the formula (10) 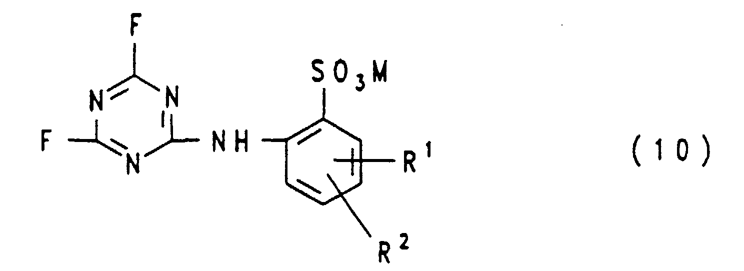 where M, R¹ and R² are as defined in claim 4 at a pH of between 4 and 7 and at a temperature of between -5°C and +20°C with a compound of the formula (11) 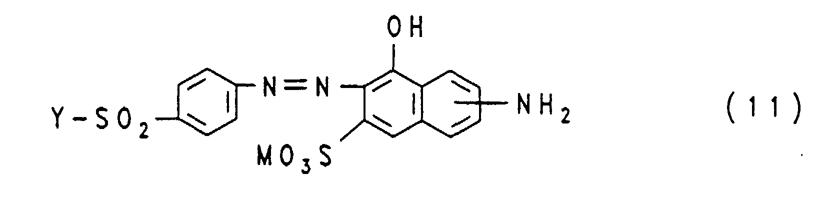 in which M and Y are as defined in claim 4 and the free amino group is attached in position 2 or 3 to the 8-hydroxy-6-sulfonaphthalene radical.

6. A method of using a monoazo compound of claim 4 for dyeing hydroxyl- and/or carboxamido-containing fiber material.

7. A process for dyeing hydroxyl- and/or carboxamido-containing fiber material in which a dye in dissolved form is applied to the material and the dye is fixed on the material by means of heat or by means of an alkali, wherein the dye is a monoazo compound of claim 4.

8. A method of using a dye mixture as claimed in at least one of claims 1 to 3 or the dyes (1) and (2) together for dyeing hydroxyl- and/or carboxamido-containing fiber material.

9. A process for dyeing hydroxyl- and/or carboxamido-containing fiber material, in which dyes in dissolved form are applied to the material and the dyes are fixed on the material by means of heat or by means of an alkali or by means of both measures, wherein as dyes a dye mixture of at least one of claims 1 to 3 or the dyes of formulae (1) and (2) of claim 1 together is or are applied and fixed.

## Revendications

1. Mélange de colorants contenant un ou plusieurs colorants disazoïques de formule générale (1) et un ou plusieurs colorants monoazoïques de formule qénérale (2) 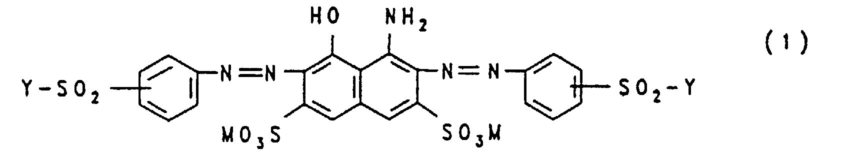 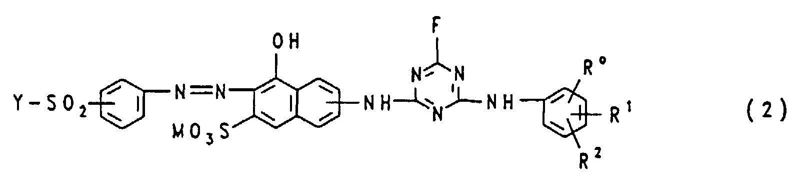 dans lesquelles :
M représente un hydrogène ou un métal alcalin ;
R⁰ représente un sulfo ou un carboxy ;
R¹ représente un hydrogène, un méthyle, un éthyle, un méthoxy, un éthoxy, un sulfo ou un carboxy ;
R² représente un hydrogène, un méthyle, un éthyle, un méthoxy ou un éthoxy ;
Y représente indépendamment, dans chaque cas, un vinyle, un β-chloroéthyle, un β-thiosulfatoéthyle, un β-phosphatoéthyle ou un β-sulfatoéthyle ;
dans la formule (2), le groupe fluorotriazinylamino est lié au radical 8-hydroxy-6-sulfonaphtalène en position 2 ou 3.

2. Mélange de colorants selon la revendication 1, caractérisé en ce que les colorants de formule générale (1) et les colorants de formule générale (2) sont présents dans le mélange en un rapport de mélange molaire de 1:0,3 à 1:1,4.

3. Mélange de colorants selon la revendication 1, caractérisé en ce que les colorants de formule générale (1) et les colorants de formule générale (2) sont présents dans le mélange en un rapport de mélange molaire de 1:0,5 à 1:1.

4. Composé monoazoique de formule générale (2A) 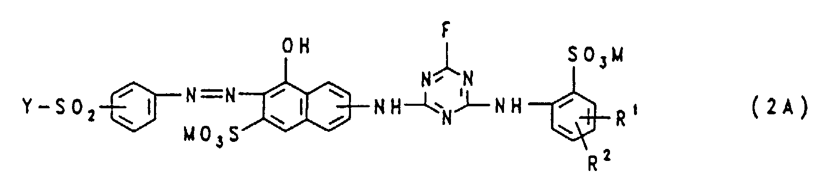 dans laquelle
M représente un hydrogène ou un métal alcalin,
Y représente un vinyle, un (3-chloroéthyle, un β-phosphatoéthyle, un β-thiosulfatoéthyle ou un β-sulfatoéthyle,
R¹ représente un hydrogène, un méthyle, un éthyle, un méthoxy, un éthoxy, un sulfo ou un carboxy,
R² représente un hydrogène, un méthyle, un éthyle, un méthoxy ou un éthoxy, et
le groupe fluorotriazinylamino est lié au radical 8-hydroxy-6-sulfonaphtalène en position 2 ou 3.

5. Procédé de préparation d'un composé monoazoïque de formule générale (2A) selon la revendication 4, caractérisé en ce que l'on procède à la diazotation d'un composé de formule générale (6) 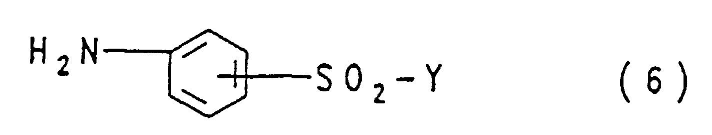 dans laquelle Y a la signification mentionnée à la revendication 4, et on procède à son couplage avec un composé de formule générale (7A) 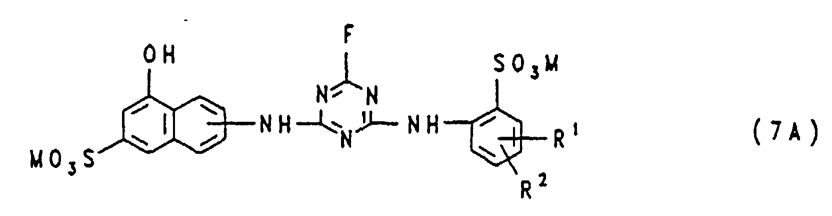 dans laquelle M, R¹ et R² ont l'une des significations indiquées à la revendication 4 et le radical fluorotriazinylamino est lié au radical 8-hydroxynaphtalène en position 2 ou 3, à un pH compris entre 3 et 7 et une température comprise entre 0 et 20°C,
ou en ce que l'on fait réagir un composé de formule générale (8) 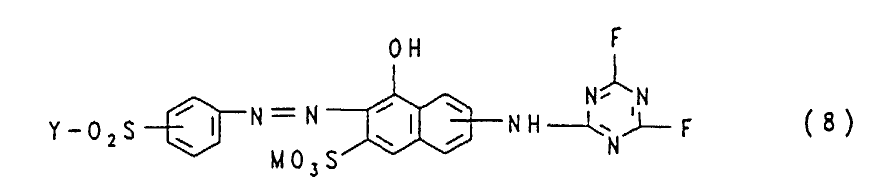 dans laquelle Y et M ont les significations mentionnées à la revendication 4 et le radical difluorotriazinylamino est lié au radical 8-hydroxy-6-sulfonaphtalène en position 2 ou 3, avec un composé de formule générale (9) 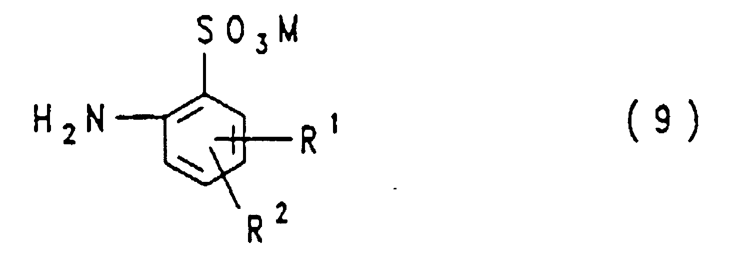 dans laquelle M, R¹ et R² ont la signification mentionnée à la revendication 4, à un pH compris entre 2 et 7 et une température comprise entre 5 et 20°C, ou en ce que l'on fait réagir un composé de formule générale (10) 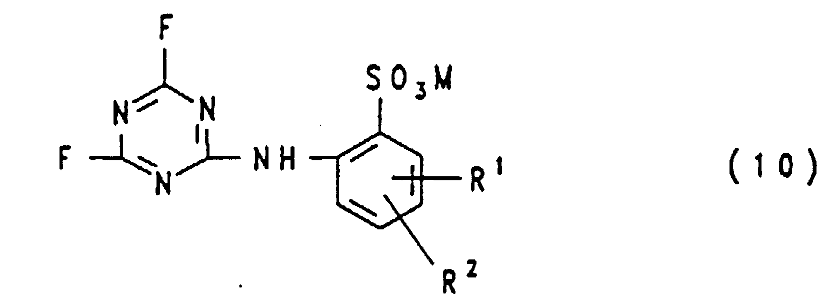 dans laquelle M, R¹ et R² ont les significations mentionnées à la revendication 4, à un pH compris entre 4 et 7 et à une température comprise entre -5°C et +20°C, avec un composé de formule générale (11) 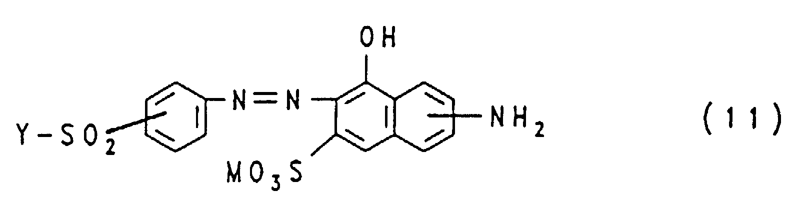 dans laquelle M et Y ont les significations mentionnées à la revendication 4 et le groupe amino libre est lié au radical 8-hydroxy-6-sulfonaphtalène en position 2 ou 3.

6. Utilisation d'un composé monoazoïque selon la revendication 4, pour la teinture d'une matière fibreuse renfermant des groupes hydroxy et/ou carboxamide.

7. Procédé de teinture d'une matière fibreuse renfermant des groupes hydroxy et/ou carboxamide, dans lequel on applique un colorant sous forme dissoute sur la matière et on fixe le colorant sur la matière par la chaleur ou à l'aide d'un agent alcalin, caractérisé en ce que le colorant est un composé monoazoïque selon la revendication 4.

8. Utilisation d'un mélange de colorants selon au moins l'une des revendications 1 à 3 ou des colorants (1) et (2) conjointement pour la teinture d'une matière fibreuse renfermant des groupes hydroxy et/ou carboxamide.

9. Procédé de teinture d'une matière fibreuse renfermant des groupes hydroxy et/ou carboxamide, dans lequel on applique des colorants sous forme dissoute sur la matière et on les fixe sur la matière par la chaleur ou à l'aide d'un agent alcalin ou au moyen des deux mesures, caractérisé en ce que l'on procède à l'application et à la fixation, en tant que colorants, d'un mélange de colorants selon au moins l'une des revendications 1 à 3 ou les colorants de formules (1) et (2) selon la revendication 1 conjointement.
